(19) Europäisches Patentamt
European Patent Office
Office européen des brevets

(11) **EP 2 301 962 B1**

(12) **EUROPÄISCHE PATENTSCHRIFT**

(45) Veröffentlichungstag und Bekanntmachung des Hinweises auf die Patenterteilung:
**30.10.2013 Patentblatt 2013/44**

(21) Anmeldenummer: **10012756.2**

(22) Anmeldetag: **26.08.2005**

(51) Int Cl.:
**C07K 14/605** (2006.01)    **C07K 14/575** (2006.01)
**A61K 38/16** (2006.01)

(54) **Erfindung betreffend GLP-1 und Exendin**

Invention affecting GLP-1 and Exendin

Invention concernant GLP-1 et l'exendine

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **03.09.2004 DE 102004043153**

(43) Veröffentlichungstag der Anmeldung:
**30.03.2011 Patentblatt 2011/13**

(62) Dokumentnummer(n) der früheren Anmeldung(en) nach Art. 76 EPÜ:
**05778889.5 / 1 784 422**

(73) Patentinhaber: **Philipps-Universität Marburg**
**35037 Marburg (DE)**

(72) Erfinder:
• **Béhé, Marti**
  **79016 Freiburg (DE)**
• **Prof. Behr, Thomas**
  **deceased (DE)**
• **Gotthardt, Martin**
  **6500 HB Nijmegen (NL)**
• **Prof. Göke, Burkhard**
  **82131 Gauting (DE)**

(74) Vertreter: **Patentanwälte Olbricht Buchhold Keulertz**
**Partnerschaft**
**Bettinastrasse 53-55**
**60325 Frankfurt (DE)**

(56) Entgegenhaltungen:
WO-A-00/69900      WO-A-91/01144
WO-A-97/46584      US-A1- 2003 073 626
US-A1- 2003 232 761

• **ENG J ET AL: "PURIFICATION AND STRUCTURE OF EXENDIN-3, A NEW PANCREATIC SECRETAGOGUE ISOLATED FROM HELODERMA HORRIDUM VENOM", JOURNAL OF BIOLOGICAL CHEMISTRY, AMERICAN SOCIETY OF BIOLOCHEMICAL BIOLOGISTS, BIRMINGHAM, US, Bd. 265, Nr. 33, 25. November 1990 (1990-11-25), Seiten 20259-20262, XP002045292, ISSN: 0021-9258**
• **STENNICKE H R ET AL: "C-terminal incorporation of fluorogenic and affinity labels using wild-type and mutagenized carboxypeptidase Y", ANALYTICAL BIOCHEMISTRY, ACADEMIC PRESS, SAN DIEGO, CA, US, Bd. 248, Nr. 1, 15. Mai 1997 (1997-05-15), Seiten 141-148, XP002298918, ISSN: 0003-2697**

**Beschreibung**

**Beschreibung und Einleitung des allgemeinen Gebietes der Erfindung**

Bei der Lokalisationsdiagnostik von gastroenteropankreatischen neuroendokrinen Tumoren wird als wichtigste diagnostische Methode neben dem Ultraschall die Somatostatin Rezeptor Szintigraphie (SRS) eingesetzt. Das Prinzip ist hier die spezifische Darstellung von Tumoren mit Hilfe von radioaktiv markierten Peptiden, die in die Tumorzellen aufgenommen werden. Sodann kann mit Hilfe von Gammakameras die Anreicherung der Radioaktivität im Tumorgewebe bildlich nachgewiesen werden. Wenn ein Tumortyp einen für die SRS erforderlichen Rezeptor z.B. für das Somatostatin-Analogon Octreotide® besitzt, ist ein Nachweis dieser Tumoren problemlos möglich. Werden entsprechende Rezeptoren jedoch nicht exprimiert, entziehen sie sich dem szintigraphischen Nachweis. Neben der Lokalisationsdiagnostik ermöglichen radioakiv markierte Peptide aber auch einen Ansatz zur Tumorbehandlung, indem man durch Markierung von Somatostatin-Analoga z.B. Octreotide® mit einem geeigneten Radionuklid ($\alpha$- oder $\beta$-Strahler) eine spezifische rezeptorgerichtete Radiopeptidtherapie durchführen kann. Da die entsprechenden Radionuklide chemisch so vom Peptid gebunden werden (z.B. über Komplexierung mit einem zuvor an das Peptid gebundenen Metallchelator), dass sie zwar in die Tumorzelle aufgenommen aber nicht mehr ausgeschleust werden können, ergibt sich eine hohe spezifische Anreicherung im Tumorgewebe.

Allerdings exprimieren eine ganze Reihe von neuroendokrinen Tumoren (NET) darunter Insulinome und kleinzellige Bronchialkarzinome nicht die erforderlichen Subtypen des Somatostatinrezeptors, die für die SRS oder die Radiopeptidtherapie mit dem Somatostatin-Analogon Octreotide® notwendig sind. Insbesondere Insulinome entziehen sich in einem erheblichen Prozentsatz der szintigraphischen Diagnostik. Auch bei kleinzelligen Bronchialkarzinomen stellt die SRS kein geeignetes Verfahren dar, da zwar die Primärtumore häufig darstellbar sind, die Metastasen jedoch nicht, da sie die Rezeptorexpression verloren haben. Somit sind sie auch keiner Radiopeptidtherapie zugänglich, die ein interessantes zusätzliches oder alternatives Therapieverfahren darstellt. Daher besteht der Bedarf an einem geeigneten Peptid, das von den genannten Tumoren aufgenommen wird.

Das Inkretinhormon Glucagon-Like Peptid-1 (GLP-1) sowie seine Analoga Exendin-3 und Exendin-4 (aus dem Speichel des Gilamonsters (Heloderma horridum und Heloderma suspectum)) sind Peptide, für die Insulinome und kleinzellige Bronchialkarzinome- neben vielen anderen Tumorarten- Rezeptoren exprimieren. Insulinome stammen von den insulinproduzierenden $\beta$-Zellen in den Langerhans'schen Inseln des Pankreas ab, in denen GLP-1 sowie Exendin-3 und Exendin-4 eine postprandiale Insulinsekretion auslösen.

**Stand der Technik**

Zur Verwendung von Glucagon-Like Peptide-1 (GLP-1) in der Szintigraphie ist eine Markierung der Peptide erforderlich. Die Verfahren dazu und zur Markierung von Proteinen mit Radionukliden sind dem Fachmann bekannt und in zahlreichen Patentschriften (z.B. DE 690 18 226 T2) und wissenschaftlichen Arbeiten belegt. Die dabei beschriebenen Peptide zur Anwendung in der diagnostischen Bildgebung und zur Vermittlung von therapeutisch wirksamen Molekülen in pathologisches Gewebe werden in der Regel am N-terminalen Ende über ein Amin in das Peptid eingeführt. Die Peptide müssen dabei bezüglich der Stabilisierung weiter modifiziert werden.

Das in der US 2003/0232761A1 verwendete GLP-1 und sein Derivat GLP-1 (7-37) sind beispielsweise am N-terminalen Ende über ein Amin modifiziert. Somit steht das N-terminale Ende von GLP-1 für die Bindung an einen GLP-1-Rezeptor nicht mehr zur Verfügung, so dass mit diesen Peptiden nur eine ungenügende Rezeptorbindung und Internalisierung möglich ist, diese Peptide also nicht zur Verwendung in der Radiopeptidtherapie von Insulinomen und kleinzelligen Bronchialkarzinomen geeignet sind. Eine Mutation z.B. ein Austausch einer Aminosäure innerhalb der Peptidsequenz von GLP-1 und Exendin-3 oder Exendin-4 und deren mögliche Modifikation durch ein Therapeutikum oder ein signalgebendes Molekül führt erfahrungsgemäß meist dazu, dass die Struktur des Peptides so gestört wird, dass keine Bindung an den Rezeptor mehr möglich ist.

Eine weitere Methode zur Modifikation von GLP-1, ohne Beeinflussung des N-Terminus ist derzeit nicht bekannt. Darüber hinaus sind keine GLP-1 Derivate bekannt, die markiert oder unmarkiert zum Einsatz in der Radiotherapie von Insulinomen und kleinzelligen Bronchialkarzinomen geeignet sind.

**Aufgabe**

Aufgabe der vorliegenden Erfindung ist daher, den Mangel im Stand der Technik zu beseitigen und Peptide bereitzustellen, die markiert werden können und auch mit dieser Markierung an den GLP-1-Rezeptor binden und die zur Herstellung eines Mittels für Diagnostik und Therapie von Erkrankungen, bei denen die GLP-1 Rezeptorexpression eine Rolle spielt, eingesetzt werden.

## Lösung der Aufgabe

Diese Aufgabe wird erfindungsgemäß gelöst durch Peptidderivate von Exendin-3, die am C-Terminus über ein Amin modifiziert sind und über den N-Terminus an den GLP-1-Rezeptor binden, und die zur Aminosäuresequenz des Peptids Exendin-3:

| H-His | Ser | Asp | Gly | Thr | Phe | Thr | Ser | Asp | Leu | Ser | Lys | Gln | Met | Glu |
|-------|-----|-----|-----|-----|-----|-----|-----|-----|-----|-----|-----|-----|-----|-----|
| 1 | 2 | 3 | 4 | 5 | 6 | 7 | 8 | 9 | 10 | 11 | 12 | 13 | 14 | 15 |
| Glu | Glu | Ala | Val | Arg | Leu | Phe | Ile | Glu | Trp | Leu | Lys | Asn | Gly | Gly |
| 16 | 17 | 18 | 19 | 20 | 21 | 22 | 23 | 24 | 25 | 26 | 27 | 28 | 29 | 30 |
| Pro | Ser | Ser | Gly | Ala | Pro | Pro | Pro | Ser | $-NH_2$ | | | | | |
| 31 | 32 | 33 | 34 | 35 | 36 | 37 | 38 | 39 | | | | | | |

eine Sequenzidentität von über 90% aufweisen, wobei die Peptidderivate eine Attachementgruppe A aufweisen, welche eine Aminosäure mit einem freien Amin oder eine organische Gruppe mit einem freien Amin ist, wobei die Attachementgruppe A am C-Terminus lokalisiert ist, wobei an die Attachementgruppe A ein Chelator zur kovalenten oder komplexen Kopplung von Radionukliden gekoppelt ist, wobei an den Chelator ein Radionuklid gekoppelt ist ausgewählt aus der Gruppe F-18, Cu-64, Cu-67, Ga-67, Ga-68, Y-86, Y-90, Tc-99m, In-111, I-123, I-124, I-131, Lu-177, Re-186, Re-188, Pt-193m, Pt-195m, Ac-225, At-211, Bi-213, Sm-153 oder Er-169.

Diese Peptidderivate werden markiert zur Herstellung eines Mittels zur Diagnostik und Therapie gut- und bösartiger Erkrankungen, bei denen die GLP-1 Rezeptorexpression eine Rolle spielt, eingesetzt.

Mit Hilfe dieser Peptidderivate von Exendin-3 wird die Herstellung eines Mittels zur szintigraphischen Anwendung realisiert, das zur Diagnostik und Therapie von GLP-1-Rezeptor exprimierenden Tumoren, darunter NET (insbesondere von Insulinomen) und kleinzelligen Bronchialkarzinomen eingesetzt wird.

Dies ist erstmals möglich, da die erfindungsgemäßen Peptidderivate über ein Amin am C-Terminus modifiziert sind, und so der N-Terminus für die Bindung an den GLP-1-Rezeptor zur Verfügung steht.

Durch die Bindung der erfindungsgemäßen radioaktiv markierten Peptidderivate von Exendin-3 an den GLP-1-Rezeptor ist die Darstellung von GLP-1-Rezeptor exprimierenden Tumoren möglich und damit erfolgt eine erhebliche Verbesserung der Patientenversorgung. In erster Linie sind NET gastroenteropankreatische NET, wie Insulinome, bei denen zur Zeit kein nicht-invasives Verfahren mit einer ausreichenden Sensitivität verfügbar ist oder im Bereich der Lunge lokalisierte kleinzellige Bronchialkarzinome, bei denen die spezifische Differenzierung zwischen entzündlichen Prozessen und Tumoren bzw. Metastasen ebenfalls derzeit durch kein nicht-invasives Verfahren möglich ist.

Weiterhin wird mittels der erfindungsgemäßen Peptidderivate die Dichte Insulinproduzierender Zellen im Pankreas dargestellt sowie die Expression von GLP-1-Rezeptoren *in vivo* und *in vitro*. Dies ist z.B. bei der Darstellung GLP-1-Rezeptoren exprimierender Zellen beim Diabetes mellitus eine *in vivo*-Darstellung, da dies die Zellen sind, die auch Insulin sezernieren. Die Darstellung der GLP-1-Rezeptordichte im Pankreas ist besonders bei Patienten mit Diabetes mellitus während und nach der Therapie mit Medikamenten wichtig. Zusätzlich wird die Verteilung von GLP-1-Rezeptoren in malignen und benignen Geweben dargestellt. Fragestellungen sind hier sowohl klinischer als auch wissenschaftlicher Natur, da es noch keine umfassenden *in vivo* Daten zur GLP-1-Rezeptorverteilung beim Menschen gibt.

Die Erfindung hat also den Vorteil, dass Peptidderivate von Exendin-3 zur Herstellung eines Mittels insbesondere zur rezeptorgerichteten spezifischen Darstellung und Therapie insbesondere von NET, hier besonders von Insulinomen und kleinzelligen Bronchialkarzinomen eingesetzt werden.

Insbesondere bei der Diagnostik von kleinzelligen Bronchialkarzinomen ist eine GLP-1-Rezeptorszintigraphie anwendbar und erlaubt erstmals die spezifische Detektion von metastatisch befallenen Lymphknoten (entzündlich veränderte Lymphknoten versus befallene Lymphknoten).

Die Verwendung der erfindungsgemäßen Peptidderivate von Exendin-3 erfolgt weiterhin als Mittel zur Diagnostik und Therapie aller gut- und bösartiger Erkrankungen, bei denen die GLP-1 Rezeptorexpression eine Rolle spielt, im Besonderen als radioaktives Mittel in der Szintigraphie (SPECT, Single Photonen Computer Tomographie) bzw. Radiopeptidtherapie und in der PET (Positronen Emissions Tomographie).

Es ist dem Fachmann leicht möglich, die Art der Markierung am C-terminalen Terminus der Peptidderivate von Exendin-3 in Abhängigkeit von der gewünschten Anwendung auszuwählen, die für die Szintigraphie bzw. Radiotherapie aus radioaktiven Nukliden besteht.

Erfindungsgemäß werden unter malignen Erkrankungen bösartige Erkrankungen verstanden, bei denen die betroffenen Gewebe Veränderungen ihres Differenzierungsgrades gegenüber gesunden Geweben aufweisen, invasives Wachstum zeigen oder deren Gewebe sich über Blut- oder Lymphbahnen ausbreitet. Hierzu gehören alle neuroendokrinen Tumoren, insbesondere die des Gastrointestinaltraktes; insbesondere auch Insulinome, Bronchialkarzinome, Pankreaskarzinom und alle anderen bösartigen Erkrankungen, die mit einer Überexpression des GLP-1-Rezeptors verbunden sind.

Erfindungsgemäß werden unter benignen Erkrankungen gutartige Erkrankungen verstanden, die sich dadurch auszeichnen, dass die betroffenen Gewebe ihren Differenzierungsgrad nicht wesentlich verlieren, kein invasives Wachstum zeigen und keine Gewebeabsiedlungen über Lymph- oder Blutstrom bilden. Hierzu gehört z.B. Diabetes mellitus aber auch Störungen des Essverhaltens oder der Psyche.

## Charakterisierung der Peptidderivate

Überraschenderweise wurde gefunden, dass Peptidderivate von Exendin-3, die am C-Terminus über Amin modifiziert sind, über einen N-Terminus an den GLP-1-Rezeptor binden. Sie zeigen sogar eine dem natürlichen Peptid ähnliche sehr hohe Affinität zum GLP-1-Rezeptor. Experimente mit tumortragenden Nacktmäusen zeigen eine spezifische Aufnahme in GLP-1-Rezeptor-positives Tumorgewebe.

Die erfindungsgemäßen Peptidderivate werden über einen Chelator am C-terminalen Amin markiert als Mittel zur Diagnostik und Therapie gut- und bösartiger Erkrankungen, bei denen die GLP-1-Rezeptorexpression eine Rolle spielt, eingesetzt. Die Art der Markierung besteht dabei insbesondere aus einem Radiometall.

Der Vorgang und die Verfahren zur Markierung sind dem Fachmann geläufig (z.B. DE 690 18 226 T2) und erfolgen durch Kopplung von Radionukliden, so dass die Rezeptorbindung oder Internalisierung der erfindungsgemäßen Peptidderivate nicht beeinträchtigt wird und der GLP-1-rezeptorbindende N-Terminus ungebunden bleibt.

Die Aminosäuresequenz des Ausgangspeptids:

**Exendin-3:**

H-His-Ser-Asp-Gly-Thr-Phe-Thr-Ser-Asp-Leu-Ser-Lys-Gln-Met-Glu-

1 2 3 4 5 6 7 8 9 10 11 12 13 14 15

Glu-Glu-Ala-Val-Arg-Leu-Phe-Ile-Glu-Trp-Leu-Lys-Asn-Gly-Gly-

16 17 18 19 20 21 22 23 24 25 26 27 28 29 30

Pro-Ser-Ser-Gly-Ala-Pro-Pro-Pro-Ser-NH$_2$

31 32 33 34 35 36 37 38 39

Erfindungsgemäß werden folgende Peptidderivate von Exendin-3 hergestellt:

## Exendin-3 (z-k)A$^{1-40}$

Wobei gilt:

z = Aminosäuren 1-38 der Exendin-3 Aminosäuresequenz
k = Aminosäuren 2-39 der Exendin-3 Aminosäuresequenz, wobei die Pepidderivate zur Aminosäuresequenz des Peptids Exendin-3 eine Sequenzidentität von über 90% aufweisen, und
A = Attachementgruppe bestehend aus einer oder mehreren Aminosäuren bzw. deren Derivate als Signalmolekül an sich oder zur Bindung von Signalmolekülen oder zur Stabilisierung. A ist bevorzugt am C-Terminus lokalisiert und ist beispielsweise ein Amin bevorzugt Lysin oder alternativ eine andere Aminosäure mit einem freien Amin wie z.B. Ornitin oder eine organischen Gruppe mit einem freien Amin an das ein Chelator zur Markierung mit Radionukliden oder ein MRT-Kontrastmittel, Fluoreszenzfarbstoff oder ein Chemotherapeutikum gekoppelt wird. Chelatoren sind beispielsweise DTPA (Diethylenetriaminepentaacetic acid, alternativ einsetzbar N, N-Bis(2-[bis(carboxymethyl) amino] -ethyl) glycine), alternativ DOTA (1, 4, 7, 10-Tetraazacyclodododecane-1, 4, 7, 10-tetra-acetic acid), HYNIC (6-Hydrazinopyridin-3-carbonsäure), MAG3 (mercaptoacetyl-glycylglycylglycine), N4 (1, 4, 8, 11-tetraazaundecane) und die bekannten Derivate aller genannten Chelatoren.

Die Hochzahl gibt an, an welcher Position innerhalb der Aminosäuresequenz sich die Attachementgruppe alternativ befinden kann.

## Exendin-3(z-k)A$^{1-40}$

hierbei sind verschieden lange Exendin-3 Derivate umfasst, wobei z die Zahlen 1 bis 38 annehmen kann, aber kleiner ist als k, welches die Zahlen 2 bis 39 annehmen kann. A ist die Attachementgruppe, die am C-Terminus steht und um eins höher ist als k. Die Attachementgruppe ist bevorzugt das Amin Lysin.

Insbesondere ist bevorzugt das Peptidderivat:

MC 13: (DTPA-Lys$^{40}$) Exendin-3 amid

Die Synthese erfolgt z.B. über Firma Peptide Specialty Laboratories GmbH nach der Methode von Merrifield und Reinigung über HPLC.

MC 13 (DTPA-Lys$^{40}$) Exendin-3 amid besteht aus der gesamten Aminosäuresequenz von Exendin-3 und trägt am C-terminalen Ende als Aminosäure mit einem freien Amin vorzugsweise ein Lysin an Position 40 sowie den Chelator DTPA.

Dem Fachmann ist ersichtlich, dass somit verschieden lange Peptidderivate von Exendin-3 vorliegen können.

Die erfindungsgemäßen Peptidderivate von Exendin-3, die am C-Terminus über ein Amin modifiziert sind und über den N-Terminus an den GLP-1-Rezeptor binden, umfassen auch Moleküle, die sich von den Aminosäuresequenzen des beschriebenen Peptids Exendin-3 an einer oder mehreren Positionen unterscheiden und einen hohen Grad an Homologie zu diesen Sequenzen aufweisen. Homologie meint dabei eine Sequenzidentität von über 90%. Die Abweichungen zu den oben beschriebenen Aminosäuresequenzen können dabei durch Deletion, Substitution und/oder Insertion entstanden sein.

## Markierung der Peptidderivate

Die erfindungsgemäßen Peptidderivate werden zur Herstellung eines Mittels zur Diagnostik und Therapie gut- und bösartiger Erkrankungen, bei denen die GLP-1 Rezeptorexpression eine Rolle spielt, in einem geeigneten Stabilisierungspuffer gelöst, z.B. zur Stabilisierung der Metalle in vorzugsweise 0, 5 M Natriumacetat pH 5, 4 mit einer Konzentration von ca. $10^{-3}$ M. Alternativ wird zur Stabilisierung von Fluoreszenzfarbstoffen ein Puffer aus Ammoniumacetat bevorzugt, zur Stabilisierung von Chemotherapeutika und Kontrastmitteln ein physiologischer Puffer.

Die Markierung erfolgt an der Attachementgruppe A durch Koppelung von Radionukliden. Dabei werden je nach Art der Anwendung für *in vivo* oder *in vitro* unterschiedliche Verfahren verwendet.

Als Radionuklide zur kovalenten oder komplexen Kopplung werden verwendet:

| Nuklid | Anwendungsverfahren | $t_{1/2}$[h] | Emittierende Strahlung | Energie[keV] | Art der Kopplung |
|---|---|---|---|---|---|
| F-18 | PET | 1,8 | $\beta^+$ | 634 | kovalent |
| Cu-64 | PET | 12,7 | $\beta^+$ | 1673 | komplex |
| Cu-67 | Therapie | 61,8 | $\beta^-$ $\gamma$ | 391 184 | komplex |
| Ga-67 | SPECT | 79,2 | $\gamma$ | 93/184/300 | komplex |
| Ga-68 | PET | 1,1 | $\beta^+$ | 2921 | komplex |
| Y-86 | PET | 14,8 | $\beta^+$ $\gamma$ | 1220 1076/1153 | komplex |
| Y-90 | Therapie | 64,1 | $\beta^-$ | 2280 | komplex |
| Tc-99m | SPECT | 6 | $\gamma$ | 140 | komplex |
| In-111 | SPECT | 67,2 | $\gamma$ | 171/245 | komplex |
| I-123 | SPECT | 13,2 | $\gamma$ | 158 | kovalent |
| I-124 | PET | 101 | $\beta^+$ $\gamma$ | 2137/1534 602 | kovalent |

(fortgesetzt)

| Nuklid | Anwendungsverfahren | $t_{1/2}$[h] | Emittierende Strahlung | Energie[keV] | Art der Kopplung |
|---|---|---|---|---|---|
| I-131 | Therapie | 192 | $\gamma$ $\beta^-$ | 364 606 | kovalent |
| Lu-177 | Therapie | 158 | $\gamma$ $\beta^-$ | 208 112/208 | komplex |
| Re-186 | Therapie | 88,8 | $\gamma$ $\beta^-$ | 137 1071 | komplex |
| Re-188 | Therapie | 17 | $\gamma$ $\beta^-$ | 155/477/632 1965/2120 | komplex |
| Pt-193m | Therapie | 104 | $\gamma$ Auger e$^-$ | 135 | komplex |
| Pt-195m | Therapie | 96 | $\gamma$ Auger e$^-$ | 98 | komplex |
| Ac-225 | Therapie | 240 | $\gamma$ $\alpha$ | 99, 150 | komplex |
| At-211 | Therapie | 7,2 | $\gamma$ Auger e$^-$ | 687 | komplex kovalent |
| Bi-213 | Therapie | 0,76 | $\gamma$ $\alpha$ | 440 | komplex |
| Sm-153 | Therapie | 46 | $\gamma$ $\beta^-$ | 103 | komplex |
| Er-169 | Therapie | 226 | $\beta^-$ | 100 | komplex |
| PET (Positronen Emissions Tomographie), SPECT (Single Photonen Computer Tomographie) | | | | | |

Chelatoren sind beispielsweise DTPA (Diethylenetriaminepentaacetic acid, N, N-Bis(2-[bis(carboxymethyl) amino] -ethyl) glycine), DOTA (1, 4, 7, 10-Tetraazacyclodododecane-1, 4, 7, 10-tetra-acetic acid), HYNIC (6-Hydrazinopyridin-3-carbonsäure), MAG3 (mercaptoacetyl-glycylglycylglycine), N4 (1, 4, 8, 11-tetraazaundecane) oder die Derivate aller genannten Chelatoren.

Je nach Art der Anwendung der erfindungsgemäßen Proteinderivate und dem aus ihnen hergestellten Mittel zur Diagnostik und Therapie gut- und bösartiger Erkrankungen, bei denen die GLP-1 Rezeptorexpression eine Rolle spielt, wird die Markierungsreaktion in zwei Varianten durchgeführt.

**Markierung zur *in vitro* Anwendung in der Radiotherapie**

3 $\mu$L der in einem geeigneten Stabilisierungspuffer vorzugsweise 0, 5 M Natriumacetat pH 5, 4 mit einer Konzentration von ca. $10^{-3}$ M gelösten erfindungsgemäßen Peptidderivate werden zur Markierung zu 500 $\mu$L 0, 5M Natriumacetat pH 5, 4 gegeben. Der pH-Wert liegt dabei zwischen 3-6. Dazu wird 185 MBq $^{111}$InCl$_3$ (Tyco, Petten, The Netherlands) in 0, 1 M HCl 500 $\mu$L hinzugefügt und während 30 Minuten bei 37°C inkubiert. Um alle Bindungsstellen abzusättigen wird nochmals 3 $\mu$L $10^{-3}$ M Lösung $^{nat}$InCl$_3$ zugegeben und nochmals 30 min inkubiert.

Die Qualitätskontrolle wird über eine HPLC- Säule vorgenommen:

Säule: CC 250/4.6 Nucleosil 120-5 C18 (Machery-Nagel, Oenisingen, Schweiz) Gradient: 0->5 min 100% 0.05 M NH$_4$OOCCH$_3$, pH 5.4 (Puffer A); 5->25 min 100% Puffer A ->50% Puffer A/50% Acetonitril.

Die Qualitätskontrolle für eine *in vitro* Anwendung wird erfüllt bei einer Markierungsausbeute von mehr als 98%.

Somit steht ein radioaktiv markiertes Mittel zur Diagnostik und Therapie gut- und bösartiger Erkrankungen, bei denen die GLP-1-Rezeptorexpression eine Rolle spielt, zur Verfügung, das beispielsweise in der Zell- und Gewebekultur mit Pankreaszellen eingesetzt wird.

**Markierung zur *in vivo* Anwendung in der Radiotherapie**

3 µL der in einem geeigneten Stabilisierungspuffer vorzugsweise 0, 5 M Natriumacetat pH 5, 4 mit einer Konzentration von ca. $10^{-3}$ M gelösten erfindungsgemäßen Peptidderivate werden zur Markierung zu 500 µL 0, 5 M Natriumacetat pH 5, 4 gegeben. Am Ende wird dazu 185 MBq $^{111}$InCl$_3$ (Tyco, Petten, The Netherlands) in 0, 1 M HCl 500 µL dazugegeben und während 30 Minuten bei 37°C inkubiert. Die Qualitätskontrolle wird über eine HPLC-Säule vorgenommen:

Säule: CC 250/4.6 Nucleosil 120-5 C18 (Machery-Nagel, Oenisingen, Schweiz) Gradient: 0->5 min 100% 0.05 M NH$_4$OOCCH$_3$, pH 5.4 (Puffer A); 5->25 min 100% Puffer A ->50% Puffer A/50% Acetonitril.

Die Qualitätskontrolle für eine *in vivo*-Anwendung wird erfüllt bei einer Markierungsausbeute von mehr als 98%.

Somit steht ein radioaktiv markiertes Mittel zur Diagnostik und Therapie gut- und bösartiger Erkrankungen, bei denen die GLP-1 Rezeptorexpression eine Rolle spielt zur Verfügung, das beispielsweise zur Tumordetektion bei Patienten eingesetzt wird.

Der Begriff Patient bezieht sich dabei gleichermaßen auf Menschen und Wirbeltiere. Damit wird das Mittel in der Human- und Veterinärmedizin verwendet. Das therapeutisch und diagnostisch wirksame Mittel der vorliegenden Erfindung wird den Patienten, als Teil einer pharmazeutisch akzeptablen Komposition entweder oral, rektal, parenteral intravenös / intrararteriell, intramuskulär, subkutan, intrathekal, intracistemal, intracraniell, intravaginal, intraperitoneal, intravasculär, lokal (Puder, Salbe oder Tropfen) oder in Sprayform verabreicht. Die entsprechende Dosierung ist je nach Anwendungsfall für Diagnostik und Therapie gut- und bösartiger Erkrankungen, bei denen die GLP-1-Rezeptorexpression eine Rolle spielt, vom Arzt festzulegen.

**Patentansprüche**

1.  Peptidderivate von Exendin-3, die am C-Terminus über ein Amin modifiziert sind und über den N-Terminus an den GLP-1-Rezeptor binden, und die zur Aminosäuresequenz des Peptids Exendin-3:

| H- His | Ser | Asp | Gly | Thr | Phe | Thr | Ser | Asp | Leu | Ser | Lys | Gln | Met | Glu |
|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 |

| Glu | Glu | Ala | Val | Arg | Leu | Phe | Ile | Glu | Trp | Leu | Lys | Asn | Gly | Gly |
|---|---|---|---|---|---|---|---|---|---|---|---|---|---|---|
| 16 | 17 | 18 | 19 | 20 | 21 | 22 | 23 | 24 | 25 | 26 | 27 | 28 | 29 | 30 |

| Pro | Ser | Ser | Gly | Ala | Pro | Pro | Pro | Ser | -NH$_2$ |
|---|---|---|---|---|---|---|---|---|---|
| 31 | 32 | 33 | 34 | 35 | 36 | 37 | 38 | 39 | |

    eine Sequenzidentität von über 90% aufweisen,
    wobei die Peptidderivate eine Attachementgruppe A aufweisen, welche eine Aminosäure mit einem freien Amin oder eine organische Gruppe mit einem freien Amin ist, wobei die Attachementgruppe A am C-Terminus lokalisiert ist, **dadurch gekennzeichnet, dass** an die Attachementgruppe A ein Chelator zur kovalenten oder komplexen Kopplung von Radionukliden gekoppelt ist, wobei an den Chelator ein Radionuklid gekoppelt ist ausgewählt aus der Gruppe F-18, Cu-64, Cu-67, Ga-67, Ga-68, Y-86, Y-90, Tc-99m, In-111, I-123, I-124, I-131, Lu-177, Re-186, Re-188, Pt-193m, Pt-195m, Ac-225, At-211, Bi-213, Sm-153 oder Er-169.

2.  Peptidderivate von Exendin-3 gemäß Anspruch 1 **dadurch gekennzeichnet, dass** die Attachementgruppe A bevorzugt Lysin ist.

3.  Peptidderivate von Exendin-3 gemäß den vorangegangenen Ansprüchen, **dadurch gekennzeichnet, dass** der Chelator ausgewählt ist aus der Gruppe DTPA (Diethylenetriaminepentaacetic acid), N, N-Bis(2-[bis(carboxymethyl) amino] -ethyl) glycine), DOTA (1, 4, 7, 10-Tetraazacyclododecane-1, 4, 7, 10-tetra-acetic acid), HYNIC (6-Hydrazinopyridin-3-carbonsäure), MAG3 (mercaptoacetyl-glycylglycylglycine), N4 (1, 4, 8, 11-tetraazaundecane) und deren bekannte Derivate.

4.  Verwendung der Peptidderivate von Exendin-3 gemäß den vorangegangenen Ansprüchen zur Herstellung eines Mittels zur Diagnostik und Therapie gut- und bösartiger Erkrankungen, bei denen die GLP-1 Rezeptorexpression eine Rolle spielt.

5.  Mittel zur Diagnostik und Therapie gut- und bösartiger Erkrankungen, bei denen die GLP-1 Rezeptorexpression eine Rolle spielt, **dadurch gekennzeichnet, dass** es markierte Peptidderivate von Exendin-3 gemäß Anspruch 1 enthält.

6.  Mittel gemäß Anspruch 5 zur rezeptorgerichteten spezifischen Darstellung und Therapie von neuroendokrinen Tumoren (NET), insbesondere Insulinomen und kleinzelligen Bronchialkarzinomen.

7.  Mittel gemäß Anspruch 5, wobei es sich bei der Diagnostik um die Szintigraphie handelt.

8. Mittel gemäß Anspruch 7, wobei es sich bei der Szintigraphie um die PET oder SPECT handelt.

**Claims**

1. Peptide derivatives of exendin-3, which derivatives are modified at the C-terminus by an amine and bind to the GLP-1 receptor by means of the N-terminus, and which derivatives exhibit a sequence identity of over 90 % to the amino acid sequence of the peptide exendin-3:

| H-His | Ser | Asp | Gly | Thr | Phe | Thr | Ser | Asp | Leu | Ser |
|-------|-----|-----|-----|-----|-----|-----|-----|-----|-----|-----|
| 1 | 2 | 3 | 4 | 5 | 6 | 7 | 8 | 9 | 10 | 11 |

| Lys | Gln | Met | Glu | Glu | Glu | Ala | Val | Arg | Leu | Phe |
|-----|-----|-----|-----|-----|-----|-----|-----|-----|-----|-----|
| 12 | 13 | 14 | 15 | 16 | 17 | 18 | 19 | 20 | 21 | 22 |

| Ile | Glu | Trp | Leu | Lys | Asn | Gly | Gly | Pro | Ser | Ser |
|-----|-----|-----|-----|-----|-----|-----|-----|-----|-----|-----|
| 23 | 24 | 25 | 26 | 27 | 28 | 29 | 30 | 31 | 32 | 33 |

| Gly | Ala | Pro | Pro | Pro | Ser | -NH$_2$; |
|-----|-----|-----|-----|-----|-----|-----|
| 34 | 35 | 36 | 37 | 38 | 39 | |

the peptide derivatives comprising an attachment group A,
which is an amino acid comprising a free amine or an organic group comprising a free amine,
the attachment group A being localised at the C-terminus,
**characterised in that**, at the attachment group A, a chelator is coupled to the covalent or complex coupling of radionuclides, a radionuclide being coupled to the chelator, which radionuclide is selected from the group comprising F-18, Cu-64, Cu-67, Ga-67, Ga-68, Y-86, Y-90, Tc-99m, In-111, I-123, I-124, I-131, Lu-177, Re-186, Re-188, Pt-193m, Pt-195m, Ac-225, At-211, Bi-213, Sm-153 or Er-169.

2. Peptide derivatives of exendin-3 according to claim 1, **characterised in that** the attachment group A is preferably lysine.

3. Peptide derivatives of exendin-3 according to any of the preceding claims, **characterised in that** the chelator is selected from the group comprising DTPA (diethylenetriaminepentaacetic acid), N, N-Bis(2-[bis(carboxymethyl) amino] -ethyl) glycine), DOTA (1, 4, 7, 10-tetraazacyclododecane-1, 4, 7, 10-tetra-acetic acid), HYNIC (6-hydrazinopyridine-3-carboxylic acid), MAG3 (mercaptoacetyl-glycylglycylglycine), N4 (1, 4, 8, 11-tetraazaundecane) and the known derivatives thereof.

4. Use of the peptide derivatives of exendin-3 according to the preceding claims for producing an agent for the diagnosis and therapy of benign and malignant diseases in which GLP-1 receptor expression plays a role.

5. Agent for the diagnosis and therapy of benign and malignant diseases, in which GLP-1 receptor expression plays a role, **characterised in that** it contains labelled peptide derivatives of exendin-3 according to claim 1.

6. Agent according to claim 5 for the receptor-targeted specific imaging and therapy of neuroendocrine tumours (NET), in particular insulinomas and small-cell bronchial carcinomas.

7. Agent according to claim 5, wherein the diagnosis is carried out by means of scintigraphy.

8. Agent according to claim 7, wherein the scintigraphy involves PET or SPECT.

**Revendications**

1. Dérivés peptidiques de l'exendine 3 qui sont modifiés en terminaison C par une amine et se lient par la terminaison N au récepteur de GLP-1 et qui présentent par rapport à la séquence d'acides aminés du peptide Exendine 3 :

| H- His | Ser | Asp | Gly | Thr | Phe | Thr | Ser | Asp | Leu | Ser | Lys | Gln | Met | Glu |
|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 |

| Glu | Glu | Ala | Val | Arg | Leu | Phe | Ile | Glu | Trp | Leu | Lys | Asn | Gly | Gly |
|---|---|---|---|---|---|---|---|---|---|---|---|---|---|---|
| 16 | 17 | 18 | 19 | 20 | 21 | 22 | 23 | 24 | 25 | 26 | 27 | 28 | 29 | 30 |

| Pro | Ser | Ser | Gly | Ala | Pro | Pro | Pro | Ser | -NH$_2$ |
|---|---|---|---|---|---|---|---|---|---|
| 31 | 32 | 33 | 34 | 35 | 36 | 37 | 38 | 39 | |

une identité de séquence de plus de 90 %,
dans lesquels les dérivés peptidiques présentent un groupe d'attachement A, lequel est un acide aminé avec une amine libre ou un groupe organique avec une amine libre,
dans lesquels le groupe d'attachement A est localisé sur la terminaison C,
**caractérisés en ce qu'**un chélateur est couplé au groupe d'attachement A pour un couplage covalent ou complexe de radionucléides, dans lesquels un radionucléide est couplé au chélateur et choisi dans le groupe comprenant F-18, Cu-64, Cu-67, Ga-67, Ga-68, Y-86, Y-90, Tc-99m, In-111, I-123, I-124, I-131, Lu-177, Re-186, Re-188, Pt-193m, Pt-195m, Ac-225, At-221, Bi-213, Sm-153 ou Er-169.

2. Dérivés peptidiques de l'exendine 3 selon la revendication 1, **caractérisés en ce que** le groupe d'attachement A est de préférence la lysine.

3. Dérivés peptidiques de l'exendine 3 selon les revendications précédentes, **caractérisés en ce que** le chélateur est choisi dans le groupe comprenant le DTPA (acide diéthylène-triaminepenta-acétique), la N, N-bis(2-[bis(carboxyméthyl) amino] -éthyl) glycine), le DOTA (acide 1, 4, 7, 10-tétra-azacyclododécane-1, 4, 7, 10-tétra-acétique), le HYNIC (acide 6-hydrazinopyridine-3-carboxylique), la MAG3 (mercapto-acétyl-glycylglycylglycine), le N4 (1, 4, 8, 11-tétra-aza-undécane) et leurs dérivés connus.

4. Utilisation de dérivés peptidiques de l'exendine 3 selon les revendications précédentes, pour la production d'un moyen de diagnostic et de traitement des maladies bénignes et malignes dans lesquelles l'expression du récepteur GLP-1 joue un rôle.

5. Moyen de diagnostic et de traitement de maladies bénignes et malignes dans lesquelles l'expression du récepteur GLP-1 joue un rôle, **caractérisé en ce qu'**il contient des dérivés peptidiques marqués de l'exendine 3 selon la revendication 1.

6. Moyen selon la revendication 5, pour la préparation spécifique dirigée sur le récepteur et le traitement des tumeurs neuro-endocrines (TNE), en particulier des insulinomes et des carcinomes bronchiques à petites cellules.

7. Moyen selon la revendication 5, dans lequel le diagnostic est la scintigraphie.

8. Moyen selon la revendication 7, dans lequel la scintigraphie est la TEP ou la TEPU.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69018226 T2
- US 20030232761 A1
- WO 1262 A
- DE 2005001503 W
- DE 102004043153

SEQUENCE LISTING

<110> Philipps-universität Marburg

<120> Invention concerning GLP-1 and exe

<130> WO 1262

<140> PCT/DE2005/001503 <141> 2005-08-26

<150> DE 102004043153.1 <151> 2004-09-03

<160> 3

<170> PatentIn version 3.4

<210> 1
<211> 37
<212> PRT
<213> Human

<220>
<221> GLP-1
<222> (1)..(37)

<300>
<302> Erfindung betreffend GLP-1 und Exendin

<310> DE 10 2004 043 153.1 <311> 2004-09-03
<312> 2006-09-03
<313> (1)..(37)

<400> 1

```
His Asp Glu Phe Glu Arg His Ala Glu Gly Thr Phe Thr Ser Asp Val
1               5               10              15

Ser Ser Tyr Leu Glu Gly Gln Ala Ala Lys Glu Phe Ile Ala Trp Leu
            20              25              30

Val Lys Gly Arg Gly
        35
```

<210> 2
<211> 39
<212> PRT
<213> human

<220>
<221> Exendin-3
<222> (1)..(39)

<300>
<302> Erfindung betreffend GLP-1 und Exendin

<310> DE 10 2004 043153.1 <311> 2004-09-03
<312> 2006-09-03
<313> (1)..(39)

<400> 2

```
His Ser Asp Gly Thr Phe Thr Ser Asp Leu Ser Lys Gln Met Glu Glu
1               5               10              15

Glu Ala Val Arg Leu Phe Ile Glu Trp Leu Lys Asn Gly Gly Pro Ser
            20              25              30

Ser Gly Ala Pro Pro Pro Ser
        35
```

<210> 3
<211> 39
<212> PRT
<213> human

<220>
<221> Exendin-4
<222> (1)..(39)

<300>
<302> Erfindung betreffend GLP-1 und Exendin

<310> DE 102004043153.1 <311> 2004-09-03
<312> 2006-09-03
<313> (1)..(39)

<400> 3

His Gly Glu Gly Thr Phe Thr Ser Asp Leu Ser Lys Gln Met Glu Glu
1               5           10          15

Glu Ala Val Arg Leu Phe Ile Glu Trp Leu Lys Asn Gly Gly Pro Ser
            20          25          30

Ser Gly Ala Pro Pro Pro Ser
35